(19) Europäisches Patentamt
European Patent Office
Office européen des brevets

(11) **EP 3 276 534 B1**

(12) **FASCICULE DE BREVET EUROPEEN**

(45) Date de publication et mention
de la délivrance du brevet:
**04.12.2024 Bulletin 2024/49**

(21) Numéro de dépôt: **17183885.7**

(22) Date de dépôt: **28.07.2017**

(51) Classification Internationale des Brevets (IPC):
**G06V 40/13** $^{(2022.01)}$    **G01J 5/34** $^{(2022.01)}$

(52) Classification Coopérative des Brevets (CPC):
**G06V 40/1306**

(54) **PROCEDE DE CAPTURE DE MOTIF THERMIQUE A CHAUFFAGE OPTIMISE DES PIXELS**

WÄRMEMUSTER-ERFASSUNGSVERFAHREN VON PIXELN MIT OPTIMIERTER ERWÄRMUNG

METHOD FOR CAPTURING A THERMAL PATTERN WITH OPTIMISED HEATING OF PIXELS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.07.2016 FR 1657390**

(43) Date de publication de la demande:
**31.01.2018 Bulletin 2018/05**

(73) Titulaires:
• **Commissariat à l'Energie Atomique et aux Energies Alternatives
75015 Paris (FR)**
• **Idemia Identity & Security France
92400 Courbevoie (FR)**

(72) Inventeurs:
• **MAINGUET, Jean-François
38100 Grenoble (FR)**
• **FOURRE, Joël Yann
78160 Marly le Roi (FR)**

(74) Mandataire: **Brevalex
Tour Trinity
1 B Place de la Défense
92400 Courbevoie (FR)**

(56) Documents cités:
**EP-A1- 0 840 250      US-A- 6 091 837
US-A1- 2006 050 935**

• **MIKI HIROFUMI: "Survey of Biometric Authentication and Proposal of and Proposal of New Sensing Mechanism", INTERNATIONAL JOURNAL OF ENGINEERING AND APPLIED SCIENCES (IJEAS), 1 April 2016 (2016-04-01), pages 2394 - 3661, XP055366924, Retrieved from the Internet <URL:https://www.ijeas.org/download_data/IJE AS0304067.pdf> [retrieved on 20170424]**
• **JEAN-FRANÇOIS MAINGUET: "Biometrics: fingerprint sensing techniques", 27 September 2014 (2014-09-27), XP055192301, Retrieved from the Internet <URL:http://web.archive.org/web/201409271040 16/http://fingerchip.pagesperso-orange.fr/biome trics/types/fingerprint_sensors_physics.htm> [retrieved on 20150529]**

EP 3 276 534 B1

**Description**

## DOMAINE TECHNIQUE ET ART ANTÉRIEUR

L'invention se rapporte à un procédé de capture de motif thermique dans lequel le chauffage des éléments de mesure thermosensible des pixels du capteur avec lequel le procédé est mis en oeuvre est optimisé. Ce procédé est avantageusement mis en oeuvre pour réaliser une capture d'empreinte digitale par détection thermique.

Un capteur d'empreintes digitales comporte des moyens de détection thermique. Ces moyens de détection thermique peuvent correspondre à des éléments pyroélectriques, des diodes, des thermistances ou tout autre élément sensible à la température permettant de convertir une variation de température en une variation de potentiel ou de courant électrique.

La détection d'une empreinte digitale peut être réalisée par des capteurs dits « passifs » exploitant une différence de températures entre le doigt et le capteur, comme décrit dans les documents US 4 394 773, US 4 429 413 et US 6 289 114. Ces capteurs ont toutefois pour inconvénient de réaliser une mesure qui dépend uniquement de la différence de températures entre le doigt et le capteur. Il peut donc arriver que le niveau du signal obtenu soit nul lorsque le doigt et le capteur sont à la même température (par exemple lorsque le doigt reste un certain temps sur le capteur), ou que le contraste des images capturées varie et pose alors des problèmes lors du traitement ultérieur des images.

Pour éliminer les problèmes soulevés par les capteurs thermiques passifs, notamment dans le cas d'une acquisition statique où le doigt ne bouge pas, des capteurs d'empreintes dits « actifs » ont été proposés, comme par exemple celui décrit dans les documents US 6 091 837 et EP 2 385 486 A1. Dans un tel capteur, chaque pixel comporte une capacité pyroélectrique formée de deux électrodes conductrices entre lesquelles une portion de matériau pyroélectrique est disposée, et un élément chauffant. Cet élément chauffant dissipe une certaine quantité de chaleur dans le pixel, et l'échauffement du pixel est mesuré au bout d'un certain temps d'acquisition, appelé temps d'intégration, en présence du doigt sur le capteur. Cela permet de distinguer, au niveau de chaque pixel, la présence d'une crête ou d'une vallée de l'empreinte détectée suivant que la chaleur est absorbée par la peau (pixel en présence d'une crête de l'empreinte) ou conservée dans le pixel (pixel en présence d'une vallée de l'empreinte). Cela conduit à une température finale plus faible dans le cas d'un pixel en présence d'une crête, où la chaleur est absorbée par la peau, par rapport à un pixel en présence d'une vallée.

Au premier ordre, un tel capteur permet de mesurer la capacité calorifique, également appelée chaleur massique ou capacité thermique massique, d'un élément en contact avec le capteur. Les mesures obtenues dépendent également de la conductivité thermique entre le capteur et la partie de l'élément (crête ou vallée dans le cas d'une empreinte digitale) en présence.

Pour former un capteur thermique actif, les pixels de ce capteur sont couplés à des éléments chauffants utilisant généralement l'effet Joule qui dissipent de la chaleur depuis un élément résistif qui est parcouru par un courant. Un des niveaux de l'empilement technologique formant les pixels est avantageusement utilisé pour former ces éléments chauffants. Par exemple, il possible d'utiliser un des niveaux électriquement conducteurs servant à réaliser les transistors et les interconnexions du capteur si l'un de ces niveaux comporte un matériau conducteur présentant une résistivité adéquate et sur lequel il suffit d'appliquer une des tensions déjà disponibles, par exemple la tension d'alimentation du capteur, pour générer un chauffage par effet Joule. Cela est notamment utilisé lorsque le capteur comporte des transistors de type TFT (« Thin-Film Transistor », ou transistor en couches minces) réalisés sur un substrat de verre ou de plastique.

Les pixels d'un tel capteur sont disposés en formant une matrice de plusieurs lignes et de plusieurs colonnes. La lecture des pixels est généralement réalisée ligne par ligne. Les éléments chauffants peuvent alors être également commandés par ligne à l'aide d'un transistor se trouvant en tête de chaque ligne, évitant ainsi l'ajout de transistors de commande dans chacun des pixels. Chaque ligne d'éléments chauffants est par exemple connectée, d'un côté de la matrice de pixels, à la masse, et de l'autre coté au transistor de commande associé à la ligne de pixels et relié à une alimentation adaptée de manière à maîtriser le courant parcourant les éléments chauffants, et donc la puissance thermique injectée par effet Joule dans les pixels par ces éléments chauffants.

Pour réaliser une lecture de la variation du nombre de charges électriques apparaissant dans les capacités pyroélectriques des pixels d'une des lignes de pixels du capteur thermique actif, la ligne d'éléments chauffants associée à la ligne de pixels destinée à être lue est activée en faisant circuler un courant de chauffage dans cette ligne d'éléments chauffants. Chacun des pixels comporte au moins un transistor de sélection, et les transistors de sélection des pixels de chaque colonne de pixels sont reliés à une ligne conductrice elle-même reliée à un circuit de lecture. Lors de la lecture d'une ligne de pixels, les transistors de sélection des pixels de cette ligne sont mis à l'état passant, ce qui permet de relier les noeuds actifs des pixels de cette ligne aux circuits de lecture se trouvant en pied de chacune des colonnes de pixels. Les transistors de sélection bloqués des pixels appartenant aux autres lignes de pixels empêchent le déplacement de charges depuis ces autres pixels vers les circuits de lecture. D'autres transistors peuvent également être présents dans chaque pixel, notamment lorsque les pixels sont lus en tension et qu'ils nécessitent la présence d'un transistor de réinitialisation et d'un transistor suiveur de tension, ou que le capteur forme également un capteur optique dans lequel un transistor est présent dans chaque pixel pour relier une photodiode de chaque pixel à un circuit de lecture dédié.

En chauffant une ligne de pixels, de la diathermie indésirable se produit entre pixels voisins, c'est-à-dire que de la chaleur passe quand même vers les pixels voisins. Cette chaleur générée se dissipe dans les pixels voisins sans être utilisée. Elle représente même un inconvénient car un temps d'attente est généralement nécessaire entre les lectures de deux lignes de pixels adjacentes afin que la chaleur générée dans la ligne de pixels voisine soit suffisamment dissipée.

De tels inconvénients se retrouvent également dans des capteurs réalisant une lecture individuelle de chaque pixel.

D'autres capteurs thermiques équipés de conducteurs chauffants ont été proposés, à l'instar de ceux décrits dans les documents de brevet EP 0840250 et US 2006/050935. Ces capteurs présentent eux aussi un effet indésirable de diathermie entre les pixels adjacents.

## EXPOSÉ DE L'INVENTION

Un but de la présente invention est de proposer un procédé de capture d'un motif thermique par un capteur à détection thermique active dans lequel le chauffage des pixels réalisé lors d'une lecture de pixels du capteur est optimisé.

La présente invention est définie par les revendications annexées.

Pour cela, la présente invention propose un procédé de capture d'un motif thermique comme défini dans la revendication 1.

Ce procédé propose de transformer le défaut lié à la diathermie en un avantage. En effet, lors de la lecture d'un ou plusieurs pixels, au lieu de chauffer uniquement ce ou ces pixels via le ou les éléments chauffants associés à ce ou ces pixels destinés à être lus, au moins un autre élément chauffant associé à au moins un autre pixel réalise également un chauffage de cet autre pixel. Ainsi, lors de la lecture du premier pixel ou du premier groupe de pixels, l'élément de mesure thermosensible du premier pixel ou de chaque pixel du premier groupe de pixels ainsi que celui du deuxième pixel ou de chaque pixel du deuxième groupe de pixels sont chauffés simultanément.

Les expressions « premier pixel » ou « premier groupe de pixels », et « deuxième pixel » ou « deuxième groupe de pixels », sont utilisée pour distinguer le ou les pixels lus vis-à-vis du ou des autres pixels qui sont également chauffés lors de la lecture.

Lorsque ce deuxième pixel, ou deuxième groupe de pixels, est adjacent au premier pixel ou au premier groupe de pixels, la chaleur générée en chauffant ce deuxième pixel ou deuxième groupe de pixels contribue également au chauffage du premier pixel ou premier groupe de pixels. Le phénomène de diathermie est donc ici utilisé judicieusement pour injecter plus de chaleur dans le pixel lu (premier pixel) et obtenir ainsi un signal de lecture délivré par le pixel lu qui soit plus important.

Deux pixels sont adjacents l'un de l'autre lorsque ces deux pixels sont disposés l'un à côté de l'autre sans aucun autre pixel disposé entre ces deux pixels adjacents. De même, deux lignes de pixels sont adjacentes l'une de l'autre lorsque ces deux lignes de pixels sont disposées l'une à côté de l'autre sans aucune autre ligne de pixels disposée entre ces deux lignes de pixels adjacentes. Enfin, deux éléments chauffants sont adjacents l'un de l'autre lorsque ces deux éléments chauffants sont disposés l'un à côté de l'autre.

Ce procédé est avantageux car il permet par exemple de dépasser, d'un point de vue chauffage, une limitation imposée sur le courant maximal de chauffage pouvant être envoyé dans les éléments chauffant car en faisant passer un courant de chauffage dans une ou plusieurs lignes adjacentes d'éléments chauffants en plus de celle associée à la ligne de pixels lue, cela permet d'augmenter la chaleur totale reçue par les capacités pyroélectriques de la ligne de pixels lue. Le courant de chauffage pouvant être envoyé dans chacune des lignes d'éléments chauffants peut être limité pour diverses raisons, par exemple parce que :

- la densité de courant devient sinon trop importante et que cela peut nuire à la fiabilité du circuit, voire mener à la fusion ou la destruction du circuit,
- les dimensions des lignes d'éléments chauffants sont limitées pour pouvoir passer d'autres lignes et/ou d'autres signaux,
- l'épaisseur des lignes d'éléments chauffants est faible en raison de diverses contraintes de fabrication,
- le matériau utilisé pour former les éléments chauffants n'accepte pas beaucoup de densité de courant.

Lorsque ce deuxième pixel ou deuxième groupe de pixels n'est pas adjacent au premier pixel ou premier groupe de pixels mais adjacent à un troisième pixel ou troisième groupe de pixels destiné à être lu après le premier pixel ou premier groupe de pixels, la chaleur générée en chauffant ce deuxième pixel ou deuxième groupe de pixels réalise un préchauffage de ce deuxième pixel ou deuxième groupe de pixels qui sera voisin du troisième pixel ou troisième groupe de pixels lu ultérieurement. Ainsi, lors de la lecture du troisième pixel ou troisième groupe de pixels, la diathermie entre les deuxième et troisième pixels ou groupes de pixels est réduite voire même supprimée du fait qu'il est possible d'avoir les deuxième et troisième pixels ou groupes de pixels à des températures proches ou similaires lors de la lecture du troisième pixel ou troisième groupe de pixels.

Ce procédé s'applique particulièrement à un capteur dans lequel les éléments de mesure thermosensible correspondent à des capacités pyroélectriques formées par des portions de matériau pyroélectrique disposées chacune entre une électrode supérieure et une électrode inférieure.

Ce procédé s'applique avantageusement à un capteur dans lequel les éléments de mesure thermosensibles sont chauffés par effet Joule par les éléments chauffants qui comportent des portions électriquement conductrices dans lesquelles un courant électrique de chauffage circule.

Les pixels d'une même ligne peuvent être lus simultanément, chaque élément chauffant pouvant être associé à une ligne de pixels et comporter une portion électriquement conductrice apte à chauffer l'élément de mesure thermosensible de chaque pixel de la ligne de pixels. Cette configuration correspond au cas d'un capteur dans lequel les lignes de pixels sont lues successivement. Dans ce cas, les premier et deuxième pixels appartiennent à des lignes de pixels distinctes.

Dans ce cas, la portion électriquement conductrice de chaque élément chauffant peut être disposée en regard des éléments de mesure thermosensible de la ligne de pixels à laquelle est associé cet élément chauffant. Dans une configuration avantageuse, les portions électriquement conductrices formant les éléments chauffants peuvent former également les électrodes supérieures des capacités pyroélectriques des pixels du capteur, c'est-à-dire les électrodes les plus proches de la surface du capteur sur laquelle se trouve l'élément dont le motif thermique est mesuré.

Un groupe de pixels peut correspondre à une ligne de pixels.

Dans cette configuration, la lecture d'une première ligne de pixels peut comporter la mise en oeuvre d'un chauffage des éléments de mesure thermosensible de cette première ligne de pixels par l'élément chauffant associé à cette première ligne de pixels et par au moins un ou deux autres éléments chauffants chacun associé à une deuxième ligne de pixels adjacente à la première ligne de pixels. Dans ce cas, les lignes d'éléments chauffants associées aux lignes de pixels voisines de la ligne de pixels lue sont utilisées pour réaliser un chauffage latéral de la ligne de pixels lue et augmenter ainsi la quantité de chaleur reçue par les pixels de la ligne lue. Un seul autre élément chauffant peut contribuer au chauffage de la ligne de pixels lue notamment lorsque la ligne de pixels lue correspond à la première ou la dernière ligne de pixels de la matrice car ces première et dernière lignes de pixels de la matrice ne comportent qu'une seule ligne de pixels voisine. Pour la lecture des autres lignes de pixels, deux autres éléments chauffants associés aux deux lignes de pixels voisines de la ligne de pixels lue peuvent contribuer au chauffage de cette ligne de pixels lue.

Le chauffage réalisé par le ou les autres éléments chauffants est mis en oeuvre tel qu'une puissance de chauffage dissipée par ce ou ces autres éléments chauffants soit différente, par exemple inférieure, de celle dissipée par l'élément chauffant associé à la première ligne de pixels. Dans ce cas, la chaleur peut être principalement apportée par l'élément chauffant associé à la ligne de pixels lue et non par le chauffage latéral réalisé par les autres éléments chauffants.

Cette maîtrise de la chaleur apportée à la ligne de pixels lue limite les effets indésirables de diathermie. En effet, un échange de chaleur entre deux éléments dépend des conductivités thermiques, mais surtout de la différence de températures entre ces éléments. En réalisant un chauffage des lignes de pixels voisines, la différence de températures entre ces lignes de pixels voisines et la première ligne de pixels est donc réduite, ce qui réduit également les échanges de chaleur entre ces lignes de pixels. La puissance de chauffage injectée dans la première ligne de pixels se dissipe donc moins dans les lignes de pixels voisines. Par contre, ce chauffage latéral augmente la consommation. Un compromis peut donc être réalisé entre le fait de chauffer plus ou moins les lignes de pixels adjacentes à la ligne de pixels lus.

En variante, chaque ligne de pixels peut être disposée entre un premier élément chauffant associé à cette ligne de pixels et un deuxième élément chauffant adjacent au premier élément chauffant, ou les éléments de mesure thermosensible de chaque ligne de pixels peuvent être disposés au moins partiellement en regard d'un premier élément chauffant associé à cette ligne de pixels et d'un deuxième élément chauffant adjacent au premier élément chauffant. De plus, la lecture de chaque ligne de pixels peut comporter un chauffage des éléments de mesure thermosensible de cette ligne de pixels par le premier élément chauffant associé à cette ligne de pixels et par le deuxième élément chauffant adjacent au premier élément chauffant.

Dans ce cas, la lecture de chaque ligne de pixels peut comporter en outre la mise en oeuvre d'un chauffage des éléments de mesure thermosensible de cette ligne de pixels par au moins un troisième élément chauffant adjacent au deuxième élément chauffant et tel que le deuxième élément chauffant soit disposé entre les premier et troisième éléments chauffants. Ainsi, un ou plusieurs éléments chauffants autres que les premier et deuxième éléments chauffants peuvent contribuer au chauffage de la ligne de pixels lue.

Le chauffage réalisé par le deuxième élément chauffant, ou par les deuxième et troisième éléments chauffants, est mis en oeuvre tel qu'une puissance de chauffage dissipée par le deuxième élément chauffant, ou par les deuxième et troisième éléments chauffants, soit différente, par exemple inférieure, de celle dissipée par le premier élément chauffant associé à la ligne de pixels. Dans ce cas, la chaleur est principalement apportée par les éléments chauffants entre lesquels se trouve la ligne de pixels lue ou par les éléments chauffants se trouvant en regard de la ligne de pixels lue, et non par le chauffage latéral réalisé par les autres éléments chauffants. Cette maîtrise de la chaleur apportée à la ligne de pixels lue limite les effets indésirables de diathermie.

La puissance de chauffage dissipée dépend notamment des caractéristiques des portions électriquement conductrices des éléments chauffants et des courants de chauffage utilisés. Par exemple, en considérant des premier et deuxième éléments chauffants comprenant des portions électriquement conductrices similaires, une puissance de chauffage dissipée par le premier élément chauffant qui est inférieure à celle dissipée par le deuxième élément chauffant peut être obtenue en faisant circuler dans le premier élément chauffant un courant de chauffage inférieur à celui circulant dans le deuxième élément chauffant.

Lors d'une séquence de lectures successives de plusieurs lignes de pixels, deux lignes de pixels lues successivement peuvent être adjacentes ou espacées l'une de l'autre par au moins une autre ligne de pixels. Lorsque les lignes de pixels lues successivement sont adjacentes, un temps de refroidissement de la ligne de pixels lue peut séparer deux lectures successives de lignes de pixels. Par contre, en espaçant les lignes de pixels lues successivement, il est possible de lire successivement deux lignes de pixels sans avoir à respecter de temps de refroidissement entre les deux lectures puisque la lecture de la ligne suivante n'est pas impactée par le chauffage réalisé lors de la lecture de la ligne précédente.

Lors d'une lecture d'une ligne de pixels, une puissance de chauffage dissipée par un élément chauffant associé à cette ligne de pixels peut être supérieure à celle dissipée par un élément chauffant associé à une ligne de pixels lue précédemment et inférieure à celle destinée à être dissipée par un élément chauffant associé à une ligne de pixels destinée à être lue ultérieurement.

Les pixels peuvent être lus individuellement les uns après les autres, et la lecture du premier pixel peut comporter un chauffage de l'élément de mesure thermosensible de ce premier pixel par l'élément chauffant associé à ce premier pixel et par au moins deux, ou quatre, autres éléments chauffants associés à au moins deux, ou quatre, deuxièmes pixels adjacents au premier pixel. Les quatre deuxièmes pixels peuvent être régulièrement répartis autour du premier pixel. Les deux ou quatre deuxièmes pixels peuvent correspondre avantageusement aux pixels voisins du premier pixel et qui se trouvent sur la même ligne ou la même colonne de pixels. D'autres pixels voisins peuvent également contribuer au chauffage du premier pixel lors de sa lecture.

L'invention porte également sur un capteur de motif thermique comme défini dans la revendication 12.

L'élément de mesure thermosensible de chaque pixel peut comprendre au moins une capacité pyroélectrique formée par au moins une portion de matériau pyroélectrique disposée entre une électrode inférieure et une électrode supérieure, l'une des électrodes inférieure et supérieure pouvant correspondre à une électrode de lecture du pixel, et l'élément chauffant associé à chaque pixel pouvant être apte à chauffer la portion de matériau pyroélectrique de la capacité pyroélectrique du pixel par effet Joule lors d'une mesure du motif thermique par la capacité pyroélectrique dudit pixel et être formé par l'autre des électrodes inférieure et supérieure.

## BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :

- la figure 1 représente une vue en coupe d'une capacité pyroélectrique d'un pixel d'un capteur de motif thermique ;
- les figures 2 à 5 représentent des vues de dessus d'une partie de capteur de motif thermique selon différents modes de réalisation ;

Des parties identiques, similaires ou équivalentes des différentes figures décrites ci-après portent les mêmes références numériques de façon à faciliter le passage d'une figure à l'autre.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

## EXPOSÉ DÉTAILLÉ DE MODES DE RÉALISATION PARTICULIERS

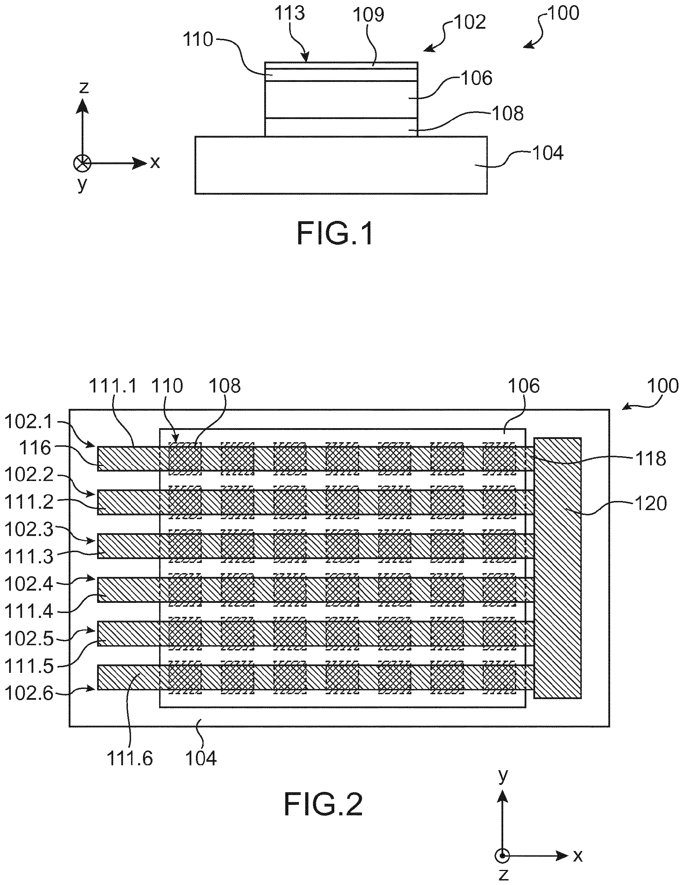

On se réfère tout d'abord à la figure 1 qui représente une vue en coupe d'une capacité pyroélectrique d'un pixel 102, qui forme l'élément de mesure thermosensible du pixel 102, d'un capteur 100 de motif thermique.

Le capteur 100 comporte un substrat 104 correspondant par exemple à un substrat de verre ou un substrat de semi-conducteur (par exemple du silicium). Un substrat 104 en verre peut être utilisé lorsque le capteur 100 est réalisé avec des transistors TFT, tandis qu'un substrat 104 en semi-conducteur, par exemple en silicium, peut être utilisé lorsque le capteur 100 comporte des transistors réalisés en technologie MOS. Le substrat 104 peut également être un substrat souple, par exemple à base de polyimide ou de PEN (polyéthylène naphtalate) ou de PET (polyéthylène téréphtalate), sur lequel les éléments électroniques du capteur 100, tels que des transistors TFT, sont réalisés par technologie électronique imprimée (par exemples via une réalisation avec des têtes d'écriture de type jet d'encre) ou par lithographie.

Les pixels 102 du capteur 100 sont disposés en formant une matrice de plusieurs lignes et plusieurs colonnes de pixels 102. Le pas des pixels 102, dans le plan (X,Y) (c'est-à-dire le plan du substrat 104), est par exemple compris entre environ 25 $\mu$m et 100 $\mu$m. Dans le cas d'un capteur 100 de résolution égale à 500 dpi (« dot per inch »), le pas des pixels 102 est égal à 50,8 $\mu$m.

Chacun des pixels 102 du capteur 100 comporte des moyens de mesure, ou de détection, thermosensible formés par une capacité pyroélectrique. Chaque capacité pyroélectrique comporte une portion 106 de matériau pyroélectrique disposée entre une électrode inférieure 108 et une électrode supérieure 110. Le matériau pyroélectrique de la portion 106 est avantageusement du P(VDF-TrFE) ou du PVDF. En variante, le matériau pyroélectrique de la portion 106 peut être de l'AIN ou du PZT, ou tout autre matériau pyroélectrique adapté pour former une capacité pyroélectrique. L'épaisseur de la portion 106 est par exemple comprise entre environ 500 nm et 10 $\mu$m.

Les électrodes 108, 110 comportent chacune au moins un matériau électriquement conducteur, par exemple un matériau métallique tel que du titane d'épaisseur égale à environ 0,2 $\mu$m, et/ou du molybdène et/ou de l'aluminium et/ou un oxyde conducteur tel que de l'ITO (oxyde d'indium et d'étain) et/ou un polymère conducteur. L'une des électrodes 108, 110, avantageusement l'électrode supérieure 110, ou chacune des deux électrodes 108, 110, peut être formée par un empilement de plusieurs matériaux électriquement conducteurs, par exemple un empilement Ti/TiN/AlCu. L'épaisseur de chacune des électrodes 108, 110 est par exemple comprise entre environ 0,1 $\mu$m et 1 $\mu$m. L'épaisseur de chacune des électrodes 108, 110 peut être plus importante, allant par exemple jusqu'à environ 5 $\mu$m, notamment lorsque ces électrodes sont réalisées par impression en utilisant des matériaux tels que l'argent, le cuivre, le carbone ou encore le PEDOT (poly(3,4-éthylènedioxythiophène).

Une couche de protection 109, correspondant par exemple à une couche de nitrure d'aluminium ou de tout autre matériau adapté à la réalisation de cette couche, recouvre l'électrode supérieure 110. L'épaisseur de la couche de protection 109 peut être comprise entre quelques microns et environ 100 $\mu$m, ou bien plus importante (par exemple de l'ordre de 300 $\mu$m ou plus). Une face supérieure 113 de la couche de protection 109 correspond à la surface au-dessus de laquelle se trouve le motif thermique destiné à être détecté, par exemple un doigt dont l'empreinte est destinée à être détectée.

Pour que le PVDF de la portion 106 acquière ses propriétés pyroélectriques (et aussi piézoélectriques), ce matériau est soumis, une fois pour toute la durée de vie de la capacité pyroélectrique, à un champ électrique d'environ 100 volts par micron d'épaisseur de PVDF. Les molécules à l'intérieur du PVDF s'orientent, et restent orientées ainsi, même lorsque le PVDF n'est plus soumis à ce champ électrique. Le PVDF peut être ainsi polarisé en appliquant une tension de polarisation initiale aux bornes des électrodes 108, 110.

Après cette polarisation initiale, lorsque la portion 106 est soumise à une variation de température $\Delta T$, cette variation de température $\Delta T$ provoque l'apparition d'un champ électrique supplémentaire générant des charges $\Delta Q$ entre les électrodes 108, 110 telles que :

$$\Delta Q = S.\gamma.\Delta T$$

Le paramètre S correspond à la surface de la portion 106 en regard de chacune des électrodes 108, 110. Le paramètre $\gamma$ correspond au coefficient pyroélectrique du matériau pyroélectrique de la portion 106. Par exemple, le coefficient pyroélectrique $\gamma$ du PVFD-TrFE est égal à environ 32 $\mu$C/m²/K.

La portion 106 et les électrodes 108, 110 formant une capacité de valeur C à laquelle s'ajoutent des capacités parasites Cp, les charges $\Delta Q$ générées induisent une différence de potentiels électriques $\Delta V$ entre les électrodes 108, 110 telle que :

$$\left(C + C_p\right)\Delta V = \Delta Q = S.\gamma.\Delta T$$

En plus des charges générées par la capacité de valeur C, d'autres charges parasites peuvent être générées également via les capacités parasites Cp présentes, par exemple celles liées à la grille du transistor de lecture dans le cas d'un circuit de lecture en tension.

Lorsque le potentiel sur l'électrode de lecture (formée par l'une des électrodes 108, 110) est fixe (lecture dite « en courant »), les charges générées s'écoulent vers le circuit de lecture en formant un courant intégré en sortie, avec dans ce cas :

$$\frac{\Delta Q}{\zeta} = \frac{S.\gamma.\Delta T}{\zeta}$$

avec $\zeta$ correspondant au temps d'intégration pendant lequel la mesure est réalisée par le pixel. Une telle lecture en courant a pour avantage d'être insensible, au premier ordre, à la valeur des capacités, en particulier des capacités parasites.

Le signe de la tension électrique $\Delta V$ obtenue entre les électrodes 108, 110, ou le sens du courant dans le cas d'une lecture en courant, dépend du sens du champ électrique avec lequel le PVDF de la portion 106 a été initialement polarisé. Dans le cas de certains autres matériaux pyroélectriques tels que le nitrure d'aluminium, ce sens de polarisation initiale dépend de la manière avec laquelle le matériau pyroélectrique a été déposé, de son ordre et de son orientation cristallographique. En outre, la tension électrique $\Delta V$ obtenue, ou le sens du courant, peut être positive ou négative suivant que la variation de température subie par la capacité pyroélectrique soit positive ou négative.

Le capteur 100 comporte également des éléments chauffants dissipant une certaine quantité de chaleur dans les pixels 102, et plus particulièrement dans la portion 106 de matériau pyroélectrique, lors de la lecture des pixels 102. Dans l'exemple de réalisation décrit ici, ces éléments chauffants sont formés directement par l'une des électrodes 108, 110 de chacune des capacités pyroélectriques. Sur l'exemple de la figure 1, l'élément chauffant de la capacité pyroélectrique du pixel 102 est formé par l'électrode supérieure 110. L'autre électrode de la capacité pyroélectrique, ici l'électrode inférieure 108, sert d'électrode de lecture du pixel 102.

Le chauffage de la portion 106 de matériau pyroélectrique est obtenu en faisant circuler un courant dans l'électrode destinée à former l'élément chauffant. Or, cette électrode sert également à la polarisation de la capacité pyroélectrique. Ainsi, la résistance de chauffage de chacun des pixels 102 sert donc également à la polarisation d'une électrode de la capacité pyroélectrique formée par la portion 106 et les électrodes 108, 110 de chacun des pixels 102 lors de la mesure réalisée par ces pixels 102 (la polarisation appliquée lors d'une mesure est différente de la polarisation initiale du PVDF précédemment décrite).

L'utilisation de l'une des électrodes 108, 110 des pixels 102 pour former les éléments chauffants est possible du fait que la valeur du potentiel électrique appliqué sur cette électrode lors d'une lecture du motif thermique est constante.

La figure 2 représente une vue de dessus de plusieurs pixels 102 du capteur 100 selon un premier mode de réalisation.

Les électrodes inférieures 108, qui correspondent aux électrodes de lecture des pixels sur lesquelles les charges générées par les capacités pyroélectriques seront récupérées pour être lues, sont formées par des premières portions de matériau électriquement conducteur distinctes et isolées électriquement les unes des autres. Ces premières portions conductrices ont chacune une section rectangulaire dans le plan du substrat 104 (plan (X,Y)), et sont disposées les unes à côté des autres sous la forme d'une matrice afin de former la matrice de pixels 102.

Le matériau pyroélectrique est réalisé sous la forme d'une seule portion 106, ou une seule couche 106, recouvrant toutes les électrodes inférieures 108.

Les électrodes supérieures 110 des pixels sont formées par plusieurs deuxièmes portions de matériau conducteur 111, référencées 111.1 à 111.6 sur la figure 2, formant chacune les électrodes supérieures 110 des pixels 102 disposés sur une même ligne. Chacune des deuxièmes portions 111 comporte une première extrémité 116 sur laquelle est appliqué un potentiel de chauffage, et une deuxième extrémité 118 reliée à une autre portion conductrice 120 commune à toutes les deuxièmes portions 111 et reliée à la masse. Les potentiels électriques appliqués sur les premières extrémités 116 des deuxièmes portions 111 sont similaires d'une portion 111 à l'autre.

Ainsi, chacune des deuxièmes portions 111.i forme un élément chauffant apte à chauffer la ligne de pixels 102.i, avec i nombre entier compris entre 1 et n (pour un capteur 100 comprenant n lignes de pixels).

Un circuit de commande (non représenté sur la figure 2) apte à commander l'allumage et l'extinction des éléments chauffants du capteur 100 comporte par exemple une liaison électrique appliquant une tension existante dans le capteur 100 sur l'extrémité 116 de chacune des deuxièmes portions 111. Par exemple, si l'extrémité 118 est reliée à la masse et qu'un potentiel de chauffage $V_{chauffe}$ est appliqué sur l'extrémité 116, un courant s'écoule alors depuis l'extrémité 116 jusqu'à l'extrémité 118 de la deuxième portion 111, provoquant un échauffement par effet Joule dans la deuxième portion 111, et échauffant ainsi la portion 106 des pixels 102 de la ligne de pixels chauffés par cette deuxième portion 111. La valeur de la tension de chauffage est choisie en fonction de la puissance de chauffage désirée, cette puissance étant fonction notamment de l'épaisseur de la portion 106 destinée à être chauffée ainsi que de l'épaisseur de la couche de protection 109, du coefficient pyroélectrique du matériau de la portions 106, de la sensibilité du circuit de lecture, du niveau de bruit du circuit de lecture et du temps d'intégration. Dans un pixel 102, la puissance de chauffage est par exemple comprise entre environ 0,1 mW et 10 mW.

Avantageusement, les deuxièmes portions de matériau conducteur 111 servant de résistance de chauffage des pixels 102 forment également les électrodes supérieures 110 des pixels 102 qui sont disposées au-dessus du matériau pyroélectrique et non les électrodes inférieures 108 car cette disposition permet de relier à la masse du capteur 100 les portions conductrices des capacités pyroélectriques qui sont le plus proches de l'extérieur du capteur 100. Une telle disposition forme une protection vis-à-vis des décharges électrostatiques (ESD) car si une telle décharge intervient, il existe alors un chemin préférentiel pour écouler les charges électriques dues à cette décharge, ce qui limite les claquages de tension avec des éléments actifs fragiles du capteur 100 tels que les transistors.

Toutefois, il est possible que les éléments chauffants soient disposés sous la portion de matériau pyroélectrique 106 et forment les électrodes inférieures 108.

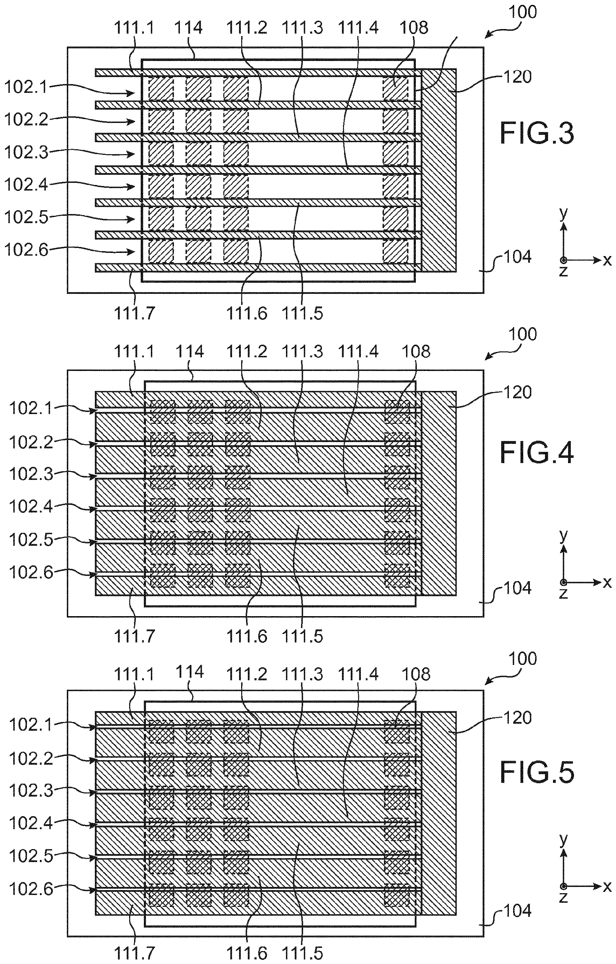

Chaque ligne de pixels 102 peut être chauffée indépendamment les unes des autres. Les valeurs des tensions de chauffage appliquées sur les deuxièmes portions 111 sont ajustées par rapport à la résistivité du métal des deuxièmes portions 111 afin de produire l'énergie thermique désirée et dissiper ainsi la puissance de chauffage souhaitée dans les pixels 102. Par exemple, il est considéré un capteur 100 comportant une matrice de 400 x 300 pixels à 500 dpi (ce qui correspond à des pixels 102 disposés les uns à côté des autres avec un pas de 50,8 $\mu$m). Pour un tel capteur 100, lorsque les deuxièmes portions 111 comportent du titane, ont chacune une épaisseur égale à environ 200 nm et présentent une résistivité de l'ordre de 2 Ohm par carré, chacune des deuxièmes portions 111 formant les électrodes supérieures 110 d'une ligne de 400 pixels ayant chacun une largeur (dimension selon l'axe Y de la figure 4, c'est-à-dire la dimension se trouvant dans le plan dans lequel s'étend la surface principale de la deuxième portion 111 et qui est perpendiculaire à la dimension principale, c'est-à-dire la longueur, de la deuxième portion 111) égale à environ 40 $\mu$m a une résistivité égale à 400 x 50/40 = 500 carrés, soit 1 kOhm. Pour obtenir une puissance dissipée par pixel 102 égale à environ 0,1 mW, soit environ 40 mW pour chaque ligne de pixels 102, un potentiel électrique d'environ 6,3 V est appliqué sur chacune des extrémités 116 des deuxièmes portions 111. Pour obtenir environ 1 mW de puissance dissipée par pixel, un potentiel électrique d'environ 20 V est appliqué sur chacune des extrémités 116 des deuxièmes portions 111.

Ainsi, l'énergie souhaitée dans chaque ligne peut être choisie en modulant la valeur de la tension ou du courant appliqué sur la ligne.

Dans l'exemple de réalisation représenté sur la figure 2, les deuxièmes portions 111 forment des bandes conductrices de largeur uniforme s'étendant chacune le long d'une ligne de pixels 102. La chaleur est ainsi diffusée de manière uniforme le long de chaque bande conductrice.

Lors d'une lecture d'une ligne de pixels, le potentiel électrique $V_{chauffe}$ appliqué sur l'une des électrodes de la capacité pyroélectrique est constant tout au long de la lecture d'un pixel. Par contre, du fait que la deuxième portion de matériau conducteur 111 sur laquelle est appliqué ce potentiel est commune à plusieurs pixels 102, la valeur du potentiel de chauffage appliqué sur l'une des électrodes des capacités pyroélectriques de chacun de ces pixels 102 est différente d'un pixel à l'autre. En considérant une ligne de pixels du capteur 100 représenté sur la figure 2, l'électrode supérieure 110 de la capacité pyroélectrique la plus proche de l'extrémité 116 est soumise à un potentiel sensiblement égal à $V_{chauffe}$. L'électrode supérieure 110 de la capacité pyroélectrique suivante est soumise à un potentiel électrique sensiblement égal à $V_{chauffe}$ - $\delta$V. Les valeurs des potentiels électriques appliqués sur les électrodes supérieures 110 des capacités pyroélectriques diminuent proportionnellement à leur éloignement vis-à-vis de l'extrémité 116. Lorsque la portion conductrice 120 est reliée à la masse, l'électrode supérieure 110 de la dernière capacité pyroélectrique, correspondant à celle qui est voisine de la portion conductrice 120, est soumise à un potentiel électrique sensiblement égal à 0V, c'est-à-dire le potentiel électrique de la masse. Cette variation du potentiel électrique de chauffage appliqué d'un pixel à l'autre ne modifie pas le chauffage réalisé d'un pixel à l'autre du fait que l'écoulement du courant dans la deuxième portion conductrice 111 provoquant l'échauffement est le même dans toute la deuxième portion conductrice 111 et le même pour tous les pixels 102 car les résistances de chauffage 128 de tous les pixels 102 sont identiques.

A la lecture des pixels de la ligne lue, les valeurs des potentiels sur chaque électrode sont différentes d'un pixel à l'autre. Par contre, pour une même variation de température, la différence de tensions, ou différence de nombre de charges, générée aux bornes des capacités pyroélectriques est identique. Or, ce sont les charges excédentaires générées par rapport à la tension de référence qui sont lues, qu'elles soient positives ou négatives.

Afin d'améliorer le chauffage des lignes de pixels, le capteur 100 réalise, lors de la lecture d'une des lignes de pixels, le chauffage de la ligne d'éléments chauffants se trouvant au-dessus de la ligne de pixels lue, mais également le chauffage d'une ou plusieurs des lignes d'éléments chauffants associés aux lignes de pixels immédiatement adjacentes. Ainsi, sur l'exemple de la figure 2, lors de la lecture de la deuxième ligne de pixels 102.2, des tensions de chauffage sont appliquées aux bornes de la deuxième portion 111.2 qui est associée à la deuxième ligne de pixels 102.2 ainsi qu'aux bornes des deuxièmes portions 111.1 et 111.3 qui sont associées aux deux lignes de pixels 102.1 et 102.3 immédiatement adjacentes à la ligne de pixels 102.1 lue. Selon un autre exemple, lors de la lecture de la quatrième ligne de pixels 102.4, des tensions de chauffage sont appliquées aux bornes des deuxièmes portions 111.3, 111.4 et 111.5.

Pour la première ligne de pixels 102.1 et la dernière ligne de pixels 102.6 qui ne comporte qu'une seule ligne de pixels adjacente, seule la ligne d'éléments chauffants associée à cette unique ligne de pixels adjacente est allumée en plus de celle associée à la ligne de pixels lue. Ainsi, lors de la lecture de la première ligne de pixels 102.1, des tensions de chauffage sont appliquées aux bornes de la deuxième portion 111.1 associée à la première ligne de pixels 102.1 ainsi qu'aux bornes de la deuxième portion 111.2 qui est associée à la deuxième ligne de pixels 102.2. De même, lors de la lecture de la dernière ligne de pixels 102.6, des tensions de chauffage sont appliquées aux bornes de la deuxième portion 111.6 qui est associée à la dernière ligne de pixels 102.6 ainsi qu'aux bornes de la deuxième portion 111.5 qui est associée à la cinquième ligne de pixels 102.5.

En variante, il est possible de réaliser le capteur 100 tel qu'il comporte une deuxième portion 111.0 supplémentaire telle que la deuxième portion 111.1 soit disposée entre la deuxième portion 111.2 et cette deuxième portion 111.0 supplémentaire (qui n'est disposée au-dessus d'aucun élément de mesure thermosensible de pixels). De même, le capteur 100 peut comporter une autre deuxième portion 111.n+1 supplémentaire telle que la deuxième portion 111.n (dans le cas d'un capteur comportant n lignes de pixels) soit disposée entre la deuxième portion 111.n-1 et cette autre deuxième portion 111.n+1 supplémentaire (qui n'est également disposée au-dessus d'aucun élément de mesure thermosensible de pixels).

La séquence de lecture des lignes de pixels du capteur 100 mise en oeuvre peut être :

- sélection de la ligne 102.i de pixels destinée à être lue, par exemple en mettant à l'état passant les transistors de sélection des pixels de la ligne 102.i ;

- allumage des éléments chauffants associés aux lignes 102.i-1, 102.i et 102.i+1 de pixels via l'application d'une tension de chauffage sur chacune des deuxièmes portions 111.i-1, 111.i et 111.i+1 associées à ces lignes de pixels ;
- réinitialisation des circuits de lecture couplés à toutes les colonnes de pixels (cette réinitialisation est réalisée après le début du chauffage des pixels pour ne pas avoir d'injection de bruit indésirable dans les circuits de lecture au moment de l'allumage du chauffage) ;
- début d'intégration des pixels de la ligne 102.i ;
- attente du temps d'intégration $\zeta$, par exemple égal à 1 ms ;
- lecture des signaux reçus par les circuits de lecture, qui correspondent aux variations de température mesurées par les pixels de la ligne 102.i qui est lue ;
- extinction des éléments chauffants ;
- désélection de la ligne 102.i.

Le paramètre « i » correspond à un nombre entier compris entre 1 et n, avec n correspondant au nombre de lignes de pixels du capteur.

De manière générale, le temps d'intégration $\zeta$ est compris entre environ 30 $\mu$s et 1ms afin d'obtenir une variation de température comprise entre environ 0,1 K et 2 K avec des injections de puissance comprises entre environ 0,1 et 1 mW par pixel.

Que la lecture soit réalisée en charges (ou en courant) ou en tension, l'allumage et l'extinction du chauffage ne sont de préférence pas réalisés pendant le temps d'intégration $\zeta$ et la lecture. En effet, l'allumage du chauffage fait varier de manière importante le potentiel d'une des électrodes des capacités pyroélectriques des pixels lus. Cette variation se retrouve donc sur l'autre électrode des capacités pyroélectriques des pixels lus. Cette variation de potentiel est, pour certains pixels, largement supérieure au signal destiné à être lu par ces pixels. Pour la même raison, il convient de limiter au maximum tous les parasites sur le potentiel $V_{chauffe}$ pendant le temps d'intégration, en particulier ceux liés aux appels de courant provenant du fonctionnement d'autres parties électroniques du capteur 100.

Après la lecture de la ligne 102.i de pixels du capteur 100, un refroidissement est réalisé pendant une certaine durée avant de lire la ligne de pixels suivante 102.i+1. Cette durée de refroidissement est par exemple comprise entre environ 3 et 5 fois le temps de chauffe de la ligne de pixels 102.i qui vient d'être lue.

Les lignes de pixels 102 du capteur 100 peuvent donc être lues successivement en partant d'une première ligne de pixels 102.1, par exemple celle se trouvant en haut du capteur 100, et en terminant par la dernière ligne de pixels 102.n, par exemple celle se trouvant en bas du capteur 100, en respectant entre les lectures de deux lignes de pixels adjacentes une certaine durée de refroidissement.

En variante, il est possible de lire successivement non pas des lignes de pixels adjacentes (c'est-à-dire la ligne 102.i, puis la ligne 102.i+1, puis la ligne 102.i+2, etc.), mais des lignes de pixels espacées les unes des autres par une ou plusieurs autres lignes de pixels. Ainsi, il est par exemple possible de lire successivement deux lignes de pixels espacées l'une de l'autre par au moins 2, 3, 4, ou plus, lignes de pixels. Par exemple, il est possible de lire successivement les lignes de pixels suivantes (les numéros représentent l'ordre des lignes de pixels, la ligne 102.1 correspondant à la ligne de pixels se trouvant en haut du capteur 100 et la ligne 102.n celle se trouvant en bas du capteur 100) : 1, 5, 10, 15, etc., puis 2, 6, 11, 16, etc. Ainsi, en choisissant de lire successivement des lignes de pixels éloignées les unes des autres, il est possible d'éviter d'attendre le refroidissement de la ligne de pixels qui vient d'être lue avant de lire la ligne de pixels suivante puisque la chaleur restante de la ligne de pixels qui vient d'être lue n'impacte pas la ligne de pixels suivante qui sera lue, du fait que cette ligne suivante n'est pas adjacente à la ligne de pixels qui vient d'être lue.

En outre, selon une variante (pouvant s'appliquer lorsque les lignes de pixels lues successivement sont adjacentes ou non), lors de la lecture de la ligne de pixels 102.i, il est possible qu'en plus du chauffage réalisé par les éléments chauffants associés aux lignes de pixels directement adjacentes (c'est-à-dire les lignes de pixels 102.i-1 et 102.i+1), une ou plusieurs autres lignes d'éléments chauffants soient allumées. Par exemple, il est possible que lors de la lecture de la ligne de pixels 102.i, les éléments chauffants associés aux lignes de pixels 102.i-2, 102.i-1, 102.i, 102.i+1 et 102.i+2 soient tous allumés, voire même un plus grand nombre de lignes d'éléments chauffants.

Le capteur 100 représenté sur la figure 2 n'est qu'un exemple de réalisation de capteur de motif thermique auquel le procédé décrit ci-dessus dans lequel des éléments de chauffage associés aux lignes de pixels adjacentes à la ligne de pixels lue sont allumés lors de la lecture de cette ligne de pixels, peut être appliqué.

La figure 3 représente un autre mode de réalisation d'un capteur 100 de motif thermique auquel ce procédé de lecture peut s'appliquer.

Contrairement au capteur 100 représenté sur la figure 2 dans lequel chacune des deuxièmes portions 111 forme à la fois les éléments chauffants et les électrodes supérieures 110 des pixels, le capteur 100 représenté sur la figure 3 comporte des éléments chauffants formés par les deuxièmes portions 111 qui ne sont pas disposées en regard des électrodes inférieures 108 et donc des éléments de mesure thermosensible des pixels. Ainsi, les électrodes supérieures des pixels 102 de ce capteur 100 sont formées par une couche électriquement conductrice 114 disposée sur le matériau pyroélectrique. Cette couche électriquement conductrice 114 est recouverte d'une couche diélectrique sur laquelle les deuxièmes portions 111 sont disposées. Cette couche électriquement conductrice 114, qui comprend par exemple une encre électriquement conductrice, forme ainsi un blindage électromagnétique entre l'élément dont le motif thermique est destiné à être détecté, par exemple un doigt dans le cas d'un capteur 100 d'empreinte digitale, et les portions 111 formant les éléments chauffants des pixels 102, évitant ainsi la récupération de bruit électromagnétique (par exemple du bruit à 50 Hz provenant du secteur) dans les mesures réalisées. Dans cette configuration, les éléments chauffants sont bien disposés au plus près de l'élément dont le motif thermique est détecté par le capteur 100.

Ainsi, sur l'exemple de la figure 3 sur lequel six lignes de pixels 102.1 à 102.6 sont représentées, chacune de ces lignes de pixels (plus précisément chacune des lignes d'électrodes supérieures de ces lignes de pixels) est disposée entre deux deuxièmes portions 111. En considérant un capteur 100 comportant n lignes de pixels, chacune des lignes de pixels 102.i est disposée entre deux deuxièmes portions 111.i et 111.i+1, c'est-à-dire entre un premier élément chauffant associé à cette ligne de pixels et un deuxième élément chauffant adjacent au premier élément chauffant.

Lors de la lecture de chacune des lignes de pixels, au moins les éléments chauffants formés par les deux deuxièmes portions 111 entre lesquelles se trouve chacune des lignes de pixels 102 sont allumés pour réaliser le chauffage de la ligne de pixels. Ainsi, pour la lecture de la ligne de pixels 102.3 (ou plus généralement 102.i), une tension de chauffage est appliquée aux extrémités de chacune des portions 111.3 et 111.4 (ou 111.i et 111.i+1).

Un nombre plus important d'éléments chauffants peuvent être allumés lors de la lecture d'une ligne de pixels du capteur 100 représenté sur la figure 3. Par exemple, lors de la lecture de la ligne de pixels 102.3 (ou plus généralement 102.i), une tension de chauffage peut être appliquée aux extrémités de chacune des portions 111.3 et 111.4 (ou 111.i et 111.i+1), mais également aux extrémités d'au moins l'une des portions 111.2 et 111.5 (ou 111.i-1 et 111.i+2), ou bien même un plus grand nombre de portions 111.

Sur l'exemple de la figure 3, les lignes d'éléments chauffants, c'est-à-dire les deuxièmes portions 111, sont disposées entre les lignes de pixels. Selon un autre exemple de réalisation du capteur tel que représenté sur la figure 4, les deuxièmes portions 111 peuvent être réalisées telles que chacune de ces deuxièmes portions 111 recouvre partiellement deux lignes de pixels adjacentes. Ainsi, sur l'exemple de la figure 4, la portion 111.2 recouvre partiellement chacune des lignes de pixels 102.1 et 102.2. De manière générale, chaque ligne de pixels 102.i est recouverte par les deuxièmes portions 111.i et 111.i+1. Les deuxièmes portions 111.1 et 111.n (correspondant à la portion 111.7 sur la figure 4) recouvrent chacune une partie d'une seule ligne de pixels.

Lors de la lecture de chacune des lignes de pixels, au moins les éléments chauffants formés par les deux deuxièmes portions 111 recouvrant la ligne de pixels lue sont allumés pour réaliser le chauffage de cette ligne de pixels. Ainsi, pour la lecture de la ligne de pixels 102.3 (ou plus généralement 102.i), une tension de chauffage est appliquée aux extrémités de chacune des portions 111.3 et 111.4 (ou 111.i et 111.i+1).

Un nombre plus important d'éléments chauffants peuvent être allumés lors de la lecture d'une ligne de pixels du capteur 100 représenté sur la figure 4. Par exemple lors de la lecture de la ligne de pixels 102.3 (ou plus généralement 102.i), une tension de chauffage peut être appliquée aux extrémités de chacune des portions 111.3 et 111.4 (ou 111.i et 111.i+1), mais également aux extrémités d'au moins l'une des portions 111.2 et 111.5 (ou 111.i-1 et 111.i+2), voire même un plus grand nombre de lignes d'éléments chauffants.

L'exemple de réalisation représenté sur la figure 4 est plus avantageux que celui représenté sur la figure 3 du fait qu'une partie de chacun des éléments chauffants est bien disposée au-dessus des pixels et non uniquement à côté des lignes de pixels comme c'est le cas sur l'exemple de réalisation de la figure 3. La chaleur générée par les éléments chauffants est ainsi mieux transmise aux éléments de mesure thermosensible des pixels.

Sur l'exemple de réalisation représenté sur la figure 4, le recouvrement de chacune des lignes de pixels 102 par les deuxièmes portions 111 est sensiblement symétrique par rapport à l'axe principal des lignes de pixels, c'est-à-dire que les surfaces de la ligne de pixels 102 se trouvant en regard de chacune des deux deuxièmes portions 111 sont sensiblement égales l'une par rapport à l'autre.

En variante, il est possible d'avoir un recouvrement des lignes de pixels 102 par les portions 111 qui soit dissymétrique, comme sur l'exemple de réalisation représenté sur la figure 5. Sur cette variante, les surfaces de la ligne de pixels 102.i se trouvant en regard de chacune des deux deuxièmes portions 111.i et 111.i+1 ne sont pas égales l'une par rapport à l'autre, et sont par exemple telles que la surface se trouvant en regard de la deuxième portion 111.i+1 soit plus importante que celle se trouvant en regard de la portion 111.i.

Pour des raisons de clarté des figures 3, 4 et 5, la portion 106 de matériau pyroélectrique n'est pas visible sur les figures 3, 4 et 5.

Dans les modes de réalisation précédemment décrits, à la lecture de chacune des lignes de pixels, plusieurs éléments chauffants sont allumés en faisant passer un même courant de chauffage dans chacun des éléments chauffants afin que ces éléments dissipent une même puissance de chauffage.

De manière avantageuse, il est possible que le circuit de commande du capteur 100 module le courant injecté dans les éléments chauffants. Par exemple, en considérant le capteur 100 précédemment décrit en lien avec la figure 2, lors de la lecture de la ligne de pixels 102.i, il est possible d'injecter dans la portion 111.i un courant de chauffage d'intensité égale $I_{chauffe}$ et d'injecter dans les portions adjacentes 111.i-1 et 111.i+1 un courant de chauffage d'intensité égale à $I_{chauffe}$ / 2. Ceci permet de maîtriser l'injection de chaleur, d'optimiser la consommation électrique pour obtenir une variation de température maximale du pixel vis-à-vis des effets indésirables de diathermie du fait que la chaleur est concentrée au niveau de la ligne de pixels lue, et que la température soit minimale sur les côtés.

De manière générale, il est possible d'injecter, lors d'une lecture d'une ligne de pixels, des courants de chauffage dans un plus grand nombre d'éléments chauffants adjacents, ces courants de chauffage ayant des intensités différentes suivant leur éloignement par rapport à la ligne de pixels lue. Le profil de la courbe formé par ces courants de chauffage injectés au sein des éléments chauffants peut correspondre sensiblement à celui d'une courbe gaussienne centrée sur l'élément chauffant associé à la ligne de pixels lue, c'est-à-dire telle que la puissance de chauffage la plus importante soit dissipée par l'élément chauffant associé à la ligne de pixels lue, et que la puissance de chauffage dissipée par les autres éléments chauffants diminue en fonction de leur éloignement de la ligne de pixels lue. Par exemple, lors de la lecture d'une ligne de pixels 102.i, il est possible d'injecter dans la portion 111.i un courant de chauffage d'intensité égale $I_{chauffe}$, d'injecter dans les portions adjacentes 111.i-1 et 111.i+1 un courant de chauffage d'intensité égale à $I_{chauffe}$ / 3, d'injecter dans les portions 111.i-2 et 111.i+2 un courant de chauffage d'intensité égale à $I_{chauffe}$ / 8, voire même d'injecter dans les portions 111.i-3 et 111.i+3 un courant de chauffage d'intensité égale à $I_{chauffe}$ / 16. Un tel profil des courants de chauffage injectés peut être symétrique ou non par rapport à l'élément chauffant associé à la ligne de pixels lue.

Différents profils de courants de chauffage injectés dans les éléments chauffants peuvent être envisagés suivant la position au sein du capteur de la ligne de pixels lue.

De plus, il est possible que les allumages des différents éléments chauffants utilisés pour la lecture d'une ligne de pixels ne soient pas simultanés, mais différés les uns des autres. Par exemple, dans le cas où les éléments chauffants 111.i-1, 111.i et 111.i+1 sont allumés lors de la lecture de la ligne de pixels 102.i, il est possible d'allumer tout d'abord l'élément chauffant 111.i, puis, par exemple 100 microsecondes plus tard, d'allumer les éléments chauffants 111.i-1 et 111.i+1.

Selon une autre variante, les courants de chauffage injectés dans les différents éléments chauffants peuvent être adaptés en fonction de l'éventuel chauffage précédemment réalisé pour un ou plusieurs de ces différentes lignes d'éléments chauffants lors d'une précédente lecture d'une ligne de pixels. Par exemple, en considérant que lors d'une lecture de la ligne de pixels 102.i, les éléments chauffants 111.i-1, 111.i et 111.i+1 ont été allumés, il est possible, lors de la lecture de la ligne de pixels suivante 102.i+1, d'injecter dans l'élément chauffant 111.i+2 un courant de chauffage C1, dans l'élément chauffant 111.i+1 un courant de chauffage C2, et dans l'élément chauffant 111.i un courant de chauffage C3, tels que C1 > C2 > C3. En effet, étant donné que l'élément chauffant 111.i+2 n'a pas servi lors de la précédente lecture de ligne de pixels, il est nécessaire d'injecter dans cet élément chauffant une puissance plus importante que celles injectées dans les éléments chauffants 111.i et 111.i+1. De plus, l'élément chauffant 111.i ayant servi à la lecture des deux précédentes lignes de pixels, celui-ci est déjà chaud et nécessite donc une puissance de chauffage moindre que celle utilisée pour l'élément chauffant 111.i+1 qui a servie uniquement à la lecture de la précédente ligne de pixels. Dans ce cas, l'intégration des pixels destinés à être lus est déjà commencée.

Il est également possible que la lecture d'une ligne de pixels 102.1 soit réalisée en deux phases : une première phase pendant laquelle seul l'élément chauffant 111.i associé à cette ligne de pixels reçoit un courant de chauffage, puis une deuxième phase pendant laquelle l'élément chauffant 111.i et un ou plusieurs autres éléments chauffants adjacents reçoivent un courant de chauffage. Un traitement postérieur des signaux de mesure obtenus lors de ces deux phases de mesure permet d'extraire l'information utile et de séparer ce qui est relatif à la ligne de pixels 102.i et renforce le signal, et ce qui est relatif plutôt aux lignes de pixels adjacentes.

Ces variantes dans lesquelles les courants de chauffage injectés ne sont pas tous les mêmes et/ou dans lesquelles les éléments chauffants ne sont pas allumés simultanément peuvent s'appliquer à tous les capteurs 100 précédemment décrits.

Dans les différents exemples de réalisation précédemment décrits, les éléments chauffants allumés parallèlement à l'élément chauffant associé à la ligne de pixels lue correspondent aux éléments chauffants qui sont adjacents à celui associé à la ligne de pixels. En variante, les éléments chauffants allumés parallèlement à l'élément chauffant associé à la ligne de pixels lue peuvent correspondre à ceux associés aux lignes de pixels adjacentes à la ligne de pixels destinée à être lue ultérieurement. Par exemple en considérant que la ligne de pixels 102.i est lue dans un premier temps, puis que la ligne de pixels 102.i+5 est lue dans un deuxième temps, puis que la ligne de pixels 102.i+10 est lue dans un troisième temps, il est possible d'allumer, lors de la lecture de la ligne de pixels 102.i, l'élément chauffant 111.i ainsi que les éléments chauffants 111.i+4 et 111.i+6. Ainsi, lors de la lecture de la ligne de pixels 102.i+5, les éléments chauffants adjacents ont été précédemment chauffés et sont donc stables thermiquement car en train de refroidir. Lors de la lecture de la ligne de pixels 102.i+5, l'élément chauffant 111.5 ainsi que les éléments chauffants 111.9 et 111.11 sont chauffés afin que lors de la prochaine lecture de la ligne de pixels 102.10, ces éléments chauffants soient déjà chauds.

Dans les modes de réalisation et variantes ci-dessus, le capteur 100 comporte des lignes de pixels et des éléments chauffants réalisant chacun un chauffage collectif de tous les pixels d'une même ligne. Toutefois, il est possible que le capteur 100 comporte des éléments chauffants pouvant chauffer les pixels individuellement, ces éléments chauffants pouvant être commandés individuellement les uns des autres. Dans un tel capteur, la lecture des pixels est réalisée non pas ligne de pixels par ligne de pixels, mais pixel par pixel. Dans ce cas, les principes précédemment exposés pour le chauffage des éléments chauffants se trouvant autour de la ligne de pixels lue s'appliquent aux pixels individuels de ce capteur. Par exemple, en considérant la lecture du pixels 102.(i,j), c'est-à-dire le pixel de la ligne i et de la colonne j du capteur 100, en plus de l'allumage de l'élément chauffant associé aux pixel 102.(i,j) lors de cette lecture, un ou plusieurs des éléments chauffants associés aux pixels se trouvant autour du pixel 102.(i,j), par exemple les éléments chauffants associés aux pixels 102.(i-1,j), 102.(i,j-1), 102.(i, j+1) et 102.(i+1,j), voire même également les éléments chauffants associés aux pixels 102.(i-1,j-1), 102.(i-1, j+1), 102.(i+1,j-1), et 102.(i+1, j+1) ou encore d'autres éléments chauffants plus éloignés du pixel lu.

Les différentes variantes précédemment décrites s'appliquent également à un tel capteur.

Dans les exemples de réalisation précédemment décrits du capteur 100, les éléments chauffants sont formés à partir d'un même niveau électriquement conducteur que celui servant à former les électrodes supérieures des capacités pyroélectriques des pixels. En variante, il est possible que les éléments chauffants ne soient pas formés à partir de ce niveau électriquement conducteur, mais à partir d'un autre niveau électriquement conducteur. Ainsi, la variante selon laquelle les électrodes supérieures sont formées par une couche électriquement conductrice distincte des éléments chauffants peut s'appliquer à tous les modes de réalisation décrits ici.

Selon une autre variante, une couche de blindage électromagnétique du capteur peut être formée en disposant sur les éléments chauffants 111 une couche diélectrique, et en disposant une couche électriquement conductrice reliée à un potentiel de référence sur cette couche diélectrique.

Bien que non représenté, le capteur 100 comporte un circuit de commande permettant d'appliquer les signaux de commande décrits ci-dessus pour piloter la lecture des pixels 102.

Le motif thermique détecté par le capteur 100 correspond avantageusement à une empreinte digitale.

Bien que non représenté, le capteur 100 peut comporter en outre un circuit électronique de traitement apte à construire une image globale du motif thermique à partir des mesures réalisées au niveau de chacun des pixels 102. Ce circuit électronique de traitement peut également être apte à comparer cette image à plusieurs images stockées dans une base de données afin d'identifier si le motif thermique détecté correspond à l'un de ceux stockés dans la base de données. Le circuit électronique de traitement peut également être apte à afficher une image du motif thermique détecté.

Dans tous les modes de réalisation et variantes précédemment décrits, le motif thermique capturé par le capteur 100 peut correspondre à celui d'une ou plusieurs empreintes digitales, mais également tout autre motif thermique. Le procédé de capture et le capteur 100 décrits précédemment peuvent notamment être adaptés pour réaliser une détection d'un motif thermique au travers d'une surface de recouvrement du capteur, par exemple lorsqu'il n'est pas nécessaire d'avoir une résolution de capture importante, tel que lors d'une détection de présence d'un élément chaud sur un support du capteur 100, par exemple la présence d'une main ou d'une autre partie d'un corps humain.

## Revendications

1. Procédé de capture d'un motif thermique par un capteur (100) comportant une matrice de plusieurs lignes et colonnes de pixels (102) et une pluralité d'éléments chauffants (111), chaque élément chauffant (111) étant associé à un pixel (102) ou à un groupe de pixels (102), et chaque élément chauffant (111) étant apte à chauffer un élément de mesure thermosensible (106) du pixel (102) ou de chaque pixel du groupe de pixels (102) indépendamment des autres éléments chauffants (111) lors d'une lecture du pixel (102) ou d'au moins un des pixels du groupe de pixels (102),

dans lequel, lors d'une étape de lecture d'un premier pixel (102) ou d'un premier groupe de pixels (102) de la matrice, un chauffage de l'élément de mesure thermosensible (106) de ce premier pixel (102) ou de chaque pixel (102) du premier groupe de pixels (102) par l'élément chauffant (111) associé à ce premier pixel (102) ou à ce premier groupe de pixels (102) et un chauffage de l'élément de mesure thermosensible (106) d'un autre pixel (102) de la matrice, appelé deuxième pixel, ou d'un deuxième groupe de pixels (102), par au moins un autre élément chauffant (111) associé à ce deuxième pixel (102) ou à ce deuxième groupe de pixels (102) qui est distinct de l'élément chauffant (111) associé au premier pixel (102) ou au premier groupe de pixels (102), sont mis en oeuvre simultanément, le deuxième pixel (102) ou le deuxième groupe de pixels (102) étant destiné à être lu avant ou après l'étape de lecture du premier pixel (102) ou du premier groupe de pixels (102), dans lequel le deuxième pixel (102) est adjacent au premier pixel (102) ou à un troisième pixel (102) destiné à être lu après le premier pixel (102) et dans lequel le courant de chauffage injecté dans les éléments chauffants est modulé tel que l'intensité du courant de chauffage injecté dans l'au moins autre élément chauffant (111) associé au deuxième pixel (102) ou au deuxième groupe de pixels (102) soit différente de l'intensité du courant de chauffage injecté dans l'élément chauffant (111) associé au premier pixel (102) ou au premier groupe de pixels (102).

2. Procédé selon la revendication 1, dans lequel les pixels (102) d'une même ligne sont lus simultanément, chaque élément chauffant (111) étant associé à une ligne de pixels (102) et comportant une portion électriquement conductrice (111) apte à chauffer l'élément de mesure thermosensible (106) de chaque pixel de la ligne de pixels (102).

3. Procédé selon la revendication 2, dans lequel la portion électriquement conductrice (111) de chaque élément chauffant est disposée en regard des éléments de mesure thermosensible (106) de la ligne de pixels (102) à laquelle est associé cet élément chauffant (111).

4. Procédé selon la revendication 3, dans lequel la lecture d'une première ligne de pixels (102) comporte la mise en oeuvre d'un chauffage des éléments de mesure thermosensible (106) de cette première ligne de pixels (102) par l'élément chauffant (111) associé à cette première ligne de pixels (102) et par au moins un ou deux autres éléments chauffants (111) chacun associé à une deuxième ligne de pixels (102) adjacente à la première ligne de pixels (102).

5. Procédé selon la revendication 4, dans lequel le chauffage réalisé par le ou les autres éléments chauffants (111) est mis en oeuvre tel qu'une puissance de chauffage dissipée par ce ou ces autres éléments chauffants (111) soit différente de celle dissipée par l'élément chauffant (111) associé à la première ligne de pixels (102).

6. Procédé selon la revendication 2, dans lequel :

   - chaque ligne de pixels (102) est disposée entre un premier élément chauffant (111) associé à cette ligne de pixels (102) et un deuxième élément chauffant (111) adjacent au premier élément chauffant (111), ou les éléments de mesure thermosensible (106) de chaque ligne de pixels (102) sont disposés au moins partiellement en regard d'un premier élément chauffant (111) associé à cette ligne de pixels (102) et d'un deuxième élément chauffant (111) adjacent au premier élément chauffant (111), et
   - la lecture de chaque ligne de pixels (102) comporte un chauffage des éléments de mesure thermosensibles (106) de cette ligne de pixels (102) par le premier élément chauffant (111) associé à cette ligne de pixels (102) et par le deuxième élément chauffant (111) adjacent au premier élément chauffant (111).

7. Procédé selon la revendication 6, dans lequel la lecture de chaque ligne de pixels (102) comporte en outre la mise en oeuvre d'un chauffage des éléments de mesure thermosensible (106) de cette ligne de pixels (102) par au moins un troisième élément chauffant (111) adjacent au deuxième élément chauffant (111) et tel que le deuxième élément chauffant (111) soit disposé entre les premier et troisième éléments chauffants (111).

8. Procédé selon l'une des revendications 6 ou 7, dans lequel le chauffage réalisé par le deuxième élément chauffant (111), ou par les deuxième et troisième éléments chauffants (111), est mis en oeuvre tel qu'une puissance de chauffage dissipée par le deuxième élément chauffant (111), ou par les deuxième et troisième éléments chauffants (111), soit différente de celle dissipée par le premier élément chauffant (111) associé à la ligne de pixels (102).

9. Procédé selon l'une des revendications 2 à 8, dans lequel, lors d'une séquence de lectures successives de plusieurs lignes de pixels (102), deux lignes de pixels (102) lues successivement sont adjacentes ou espacées l'une de l'autre par au moins une autre ligne de pixels (102).

10. Procédé selon l'une des revendications 4 ou 7, dans lequel, lors d'une lecture d'une ligne de pixels (102), une puissance de chauffage dissipée par un élément chauffant (111) associé à cette ligne de pixels (102) est supérieure à celle dissipée par un élément chauffant (111) associé à une ligne de pixels (102) lue précédemment et inférieure à celle destinée à être dissipée par un élément chauffant (111) associé à une ligne de pixels (102) destinée à être lue ultérieurement.

11. Procédé selon la revendication 1, dans lequel les pixels (102) sont lus individuellement les uns après les autres, et dans lequel la lecture du premier pixel (102) comporte un chauffage de l'élément de mesure thermosensible (106) de ce premier pixel (102) par l'élément chauffant (111) associé à ce premier pixel (102) et par au moins deux autres éléments chauffants (111) associés à au moins deux deuxièmes pixels (102) adjacents au premier pixel (102).

12. Capteur de motif thermique (100) comportant une matrice de plusieurs lignes et colonnes de pixels (102) et une pluralité d'éléments chauffants (111), chaque élément chauffant (111) étant associé à un pixel (102) ou à un groupe de pixels (102), et chaque élément chauffant (111) étant apte à chauffer un élément de mesure thermosensible (106) du pixel (102) ou de chaque pixel du groupe de pixels (102) indépendamment des autres éléments chauffants (111) lors d'une lecture du pixel (102) ou d'au moins un des pixels du groupe de pixels (102), et comportant en outre un circuit de commande apte à commander l'allumage et l'extinction des éléments chauffants (111) et à mettre en oeuvre un procédé de capture d'un motif thermique selon l'une des revendications précédentes.

13. Capteur de motif thermique (100) selon la revendication 12, dans lequel l'élément de mesure thermosensible (106) de chaque pixel (102) comprend au moins une capacité pyroélectrique formée par au moins une portion de matériau pyroélectrique (106) disposée entre une électrode inférieure (108) et une électrode supérieure (111), dans lequel l'une des électrodes inférieure (108) et supérieure correspond à une électrode de lecture du pixel (102) et dans lequel l'élément chauffant (111) associé à chaque pixel (102) est apte à chauffer la portion de matériau pyroélectrique (106) de la capacité pyroélectrique du pixel (102) par effet Joule lors d'une mesure du motif thermique par la capacité pyroélectrique dudit pixel (102) et est formé par l'autre des électrodes inférieure et supérieure (111).


**Patentansprüche**

1. Verfahren zum Erfassen eines Wärmemusters durch einen Sensor (100), eine Matrix aus mehreren Zeilen und Spalten von Pixeln (102) und eine Vielzahl von Heizelementen (111) enthaltend, wobei jedes Heizelement (111) einem Pixel (102) oder einer Gruppe von Pixeln (102) zugeordnet ist, und jedes Heizelement (111) dazu geeignet ist, ein wärmeempfindliches Messelement (106) des Pixels (102) oder jedes Pixels der Gruppe von Pixeln (102) zu erwärmen, unabhängig von den anderen Heizelementen (111), beim Lesen des Pixels (102) oder mindestens eines der Pixel der Gruppe von Pixeln (102),

    wobei, in einem Schritt des Lesens eines ersten Pixels (102) oder einer ersten Gruppe von Pixeln (102) der Matrix, eine Erwärmung des wärmeempfindlichen Messelements (106) dieses ersten Pixels (102) oder jedes Pixels (102) der ersten Gruppe von Pixeln (102) durch das Heizelement (111), das diesem ersten Pixel (102) oder dieser ersten Gruppe von Pixeln (102) zugeordnet ist, und eine Erwärmung des wärmeempfindlichen Messelements (106) eines anderen Pixels (102) der Matrix, zweites Pixel genannt, oder von einer zweiten Gruppe von Pixeln (102), durch mindestens ein anderes Heizelement (111), das diesem zweiten Pixel (102) oder dieser zweiten Gruppe von Pixeln (102) zugeordnet ist, die separat vom Heizelement (111) sind, das dem ersten Pixel (102) oder der ersten Gruppe von Pixeln (102) zugeordnet ist, gleichzeitig implementiert werden, wobei das zweite Pixel (102) oder die zweite Gruppe von Pixeln (102) dazu bestimmt sind, vor oder nach dem Schritt der Lesung des ersten Pixels (102) oder der ersten Gruppe von Pixeln (102) gelesen zu werden,
wobei das zweite Pixel (102) benachbart zum ersten Pixel (102) ist oder zu einem dritten Pixel (102), das dazu bestimmt ist, nach dem ersten Pixel (102) gelesen zu werden,
und wobei der Heizstrom, der in die Heizelemente eingespeist wird, so moduliert ist, dass sich die Stärke des Heizstroms, der in das mindestens andere Heizelement (111) eingespeist wird, das dem zweiten Pixel (102) oder der zweiten Gruppe von Pixeln (102) zugeordnet ist, von der Stärke des Heizstroms unterscheidet, der in das Heizelement (111) eingespeist wird, das dem ersten Pixel (102) oder der ersten Gruppe von Pixeln (102) zugeordnet ist.

2. Verfahren nach Anspruch 1, wobei die Pixel (102) einer selben Zeile gleichzeitig gelesen werden, wobei jedes Heizelement (111) einer Pixelzeile (102) zugeordnet ist und einen elektrisch leitenden Teil (111) enthält, der dazu geeignet ist, das wärmeempfindliche Messelement (106) jedes Pixels der Pixelzeile (102) zu erwärmen.

3. Verfahren nach Anspruch 2, wobei der elektrisch leitende Teil (111) jedes Heizelements gegenüber den wärmeempfindlichen Messelementen (106) der Pixelzeile (102), der dieses Heizelement (111) zugeordnet ist, angeordnet ist.

4. Verfahren nach Anspruch 3, wobei das Lesen einer ersten Pixelzeile (102) die Implementierung einer Erwärmung der wärmeempfindlichen Messelemente (106) dieser ersten Pixelzeile (102) durch das Heizelement (111), das dieser ersten Pixelzeile (102) zugeordnet ist, enthält, und durch mindestens ein oder zwei weitere Heizelemente (111), die jeweils einer zweiten Pixelzeile (102) zugeordnet sind, die zur ersten Pixelzeile (102) benachbart ist.

5. Verfahren nach Anspruch 4, wobei die durch das oder die andere(n) Heizelement(e) (111) realisierte Erwärmung so implementiert wird, dass sich eine durch das oder die andere(n) Heizelement(e) (111) abgegebene Heizleistung von der unterscheidet, die durch das Heizelement (111) abgegeben wird, das der ersten Pixelzeile (102) zugeordnete ist.

**6.** Verfahren nach Anspruch 2, wobei:

- jede Pixelzeile (102) zwischen einem ersten Heizelement (111), das dieser Pixelzeile (102) zugeordnet ist, und einem zweiten Heizelement (111), das zum ersten Heizelement (111) benachbart ist, angeordnet ist, oder die wärme-empfindlichen Messelemente (106) jeder Pixel-zeile (102) mindestens teilweise gegenüber einem ersten Heizelement (111) angeordnet sind, das dieser Pixelzeile (102) zugeordnet ist, und einem zweiten Heizelement (111), das zum ersten Heizelement (111) benachbart ist, und
- das Lesen jeder Pixelzeile (102) eine Erwärmung der wärmeempfindlichen Messelemente (106) dieser Pixelzeile (102) durch das erste Heizelement (111), das dieser Pixelzeile (102) zugeordnet ist, enthält, und durch das zweite Heizelement (111), das zum ersten Heizelement (111) benachbart ist.

**7.** Verfahren nach Anspruch 6, wobei das Lesen jeder Pixelzeile (102) ferner die Implementierung einer Erwärmung der wärmeempfindlichen Messelemente (106) dieser Pixelzeile (102) durch mindestens ein drittes Heizelement (111), das zum zweiten Heizelement (111) benachbart ist, enthält, und so, dass das zweite Heizelement (111) zwischen dem ersten und dritten Heizelement (111) angeordnet ist.

**8.** Verfahren nach einem der Ansprüche 6 oder 7, wobei die Erwärmung, die durch das zweite Heizelement (111) realisiert wird oder durch das zweite und dritte Heizelement (111), so implementiert wird, dass eine Heizleistung, die durch das zweite Heizelement (111) abgegeben wird oder durch das zweite und dritte Heizelement (111), sich von derjenigen unterscheidet, die durch das erste Heizelement (111), das der Pixelzeile (102) zugeordnet ist, abgegeben wird.

**9.** Verfahren nach einem der Ansprüche 2 bis 8, wobei, bei einer Folge aufeinanderfolgender Lesevorgänge mehrerer Pixelzeilen (102), zwei Pixelzeilen (102), die nacheinander gelesen werden, benachbart sind oder durch mindestens eine andere Pixelzeile (102) voneinander beabstandet sind.

**10.** Verfahren nach einem der Ansprüche 4 oder 7, wobei, beim Lesen einer Pixelzeile (102), eine Heizleistung, die von einem Heizelement (111) abgegeben wird, das dieser Pixelzeile (102) zugeordnet ist, größer ist als die, die von einem Heizelement (111) abgegeben wird, das einer Pixelzeile (102) zugeordnet ist, die zuvor gelesen wurde, und geringer ist als die, die dazu bestimmt ist, von einem Heizelement (111) abgegeben zu werden, das einer Pixelzeile (102) zugeordnet ist, die dazu bestimmt ist, später gelesen zu werden.

**11.** Verfahren nach Anspruch 1, wobei die Pixel (102) einzeln nacheinander gelesen werden, und wobei das Lesen des ersten Pixels (102) eine Erwärmung des wärmeempfindlichen Messelements (106) dieses ersten Pixels (102) durch das Heizelement (111) enthält, das diesem ersten Pixel (102) zugeordnet ist, und durch mindestens zwei andere Heizelemente (111), die mindestens zwei zweiten Pixeln (102) zugeordnet sind, die zum ersten Pixel (102) benachbart sind.

**12.** Wärmemustersensor (100), eine Matrix aus mehreren Zeilen und Spalten von Pixeln (102) und einer Vielzahl von Heizelementen (111) enthaltend, wobei jedes Heizelement (111) einem Pixel (102) oder einer Gruppe von Pixeln (102) zugeordnet ist, und jedes Heizelement (111) dazu geeignet ist, ein wärmeempfindliches Messelement (106) des Pixels (102) oder jedes Pixels der Gruppe von Pixeln (102) zu erwärmen, unabhängig von den anderen Heizelementen (111), beim Lesen des Pixels (102) oder mindestens eines der Pixel der Gruppe von Pixeln (102), und ferner einen Steuerschaltkreis enthält, der dazu geeignet ist, das Einschalten und das Ausschalten der Heizelemente (111) zu steuern, und ein Verfahren zum Erfassen eines Wärmemusters nach einem der vorhergehenden Ansprüche zu implementieren.

**13.** Wärmemustersensor (100) nach Anspruch 12, wobei das wärmeempfindliche Messelement (106) jedes Pixels (102) mindestens eine pyroelektrische Kapazität umfasst, die aus mindestens einem Teil aus pyroelektrischem Material (106) gebildet ist, der zwischen einer unteren Elektrode (108) und einer oberen Elektrode (111) angeordnet ist, wobei eine der unteren (108) und oberen Elektroden einer Elektrode der Lesung des Pixels (102) entspricht und das Heizelement (111), das jedem Pixel (102) zugeordnet ist, dazu geeignet ist, den Teil aus pyroelektrischem Material (106) der pyroelektrischen Kapazität des Pixels (102) durch Joule-Effekt bei einer Messung des Wärmemusters durch die pyroelektrische Kapazität des Pixels (102) zu erwärmen, und durch die andere der unteren und oberen Elektroden (111) gebildet wird.

**Claims**

**1.** A method of capturing a thermal pattern by a sensor (100) comprising a matrix of several rows and columns of pixels (102) and a plurality of heating elements (111), each heating element (111) being associated with a pixel (102) or a group of pixels (102), and each heating element (111) being capable of heating a heat sensitive measurement element (106) for measurement of the pixel (102) or each pixel in the group of pixels (102) independently of the other heating elements (111) during reading of the pixel (102) or of at least one of the pixels in the group of pixels (102),

in which, during a reading of a first pixel (102) or of a first group of pixels (102), the heat sensitive measurement element (106) of this first pixel (102) or of each pixel (102) of the first group of pixels (102) is heated by the heating element (111) associated with this first pixel (102) or with this first group of pixels (102), and the heat sensitive measurement element (106) of another pixel (102) called second pixel, or of a second group of pixels (102), in the matrix is heated simultaneously by at least one other heating element (111) associated with this second pixel (102) or with this second group of pixels (102), the at least one other heating element (111) being distinct from the heating element (111) associated with the first pixel (102) or with the first group of pixel (102), the second pixel (102) or the second group of pixels (102) being intended to be read before or after the reading of the first pixel (102) or of the first group of pixels (102), in which the second pixel (102) is adjacent to the first pixel (102) or to a third pixel (103) being intended to be read after the first pixel (102) and in which the heating current injected into the heating elements is modulated so that the intensity of the heating current, injected into the at least one other heating element (111) associated with the second pixel (102) or with the second group of pixels (102), is different from the intensity of the heating current injected into the heating element (111) associated with the first pixel (102) or with the first group of pixels (102).

2. The method according to claim 1, in which the pixels (102) in a particular row are read simultaneously, each heating element (111) being associated with a row of pixels (102) and comprising an electrically conducting portion (111) capable of heating the heat sensitive measurement element (106) of each pixel in the row of pixels (102).

3. The method according to claim 2, in which the electrically conducting portion (111) of each heating element is positioned facing the heat sensitive measurement elements (106) of the row of pixels (102) with which this heating element (111) is associated.

4. The method according to claim 3, in which reading of a first row of pixels (102) includes the use of heating of heat sensitive measurement elements (106) of this first row of pixels (102) by the heating element (111) associated with this first row of pixels (102) and by at least one or two other heating elements (111) each associated with a second row of pixels (102) adjacent to the first row of pixels (102).

5. The method according to claim 4, in which heating applied by the other heating element (111) or by the other heating elements (111) is implemented such that a heating power dissipated by this or these other heating elements (111) is different from the heating power dissipated by the heating element (111) associated with the first row of pixels (102).

6. The method according to claim 2, in which:

- each row of pixels (102) is located between a first heating element (111) associated with this row of pixels and a second heating element (111) adjacent to the first heating element (111), or the heat sensitive measurement elements (106) of each row of pixels (102) are at least partially located facing a first heating element (111) associated with this row of pixels (102) and a second heating element (111) adjacent to the first heating element (111), and
- reading of each row of pixels (102) includes heating of the heat sensitive measurement elements (106) of this row of pixels (102) by the first heating element (111) associated with this row of pixels (102) and by the second heating element (111) adjacent to the first heating element (111).

7. The method according to claim 6, in which reading of each row of pixels (102) also includes heating of the heat sensitive measurement elements (106) in this pixel row by at least one third heating element (111) adjacent to the second heating element (111) and such that the second heating element (111) is located between the first and third heating elements (111).

8. The method according to claim 6 or 7, in which heating applied by the second heating element (111), or by the second and the third heating elements (111) is applied such that a heating power dissipated by the second heating element (111) or by the second and third heating elements (111) is different from the heating power dissipated by the first heating element (111) associated with the row of pixels (102).

9. The method according to any of claims 2 to 8, in which during a sequence of successive reads of several rows of pixels (102), two rows of successively read pixels (102) are adjacent or separated from each other by at least one other row of pixels.

10. The method according to claim 4 or 7, in which, when a row of pixels (102) is being read, a heating power dissipated by a heating element (111) associated with this row of pixels (102) is higher than the heating power dissipated by a heating element (111) associated with a previously read row of pixels (102) and less than the heating power that will be dissipated by a heating element (111) associated with a row of pixels (102) to be read later.

11. The method according to claim 1, in which pixels (102) are read individually one by one, and in which reading of the first pixel (102) includes heating of the heat sensitive measurement element (106) of this first pixel (102) by the heating element (111) associated with this first pixel (102) and by at least two other heating elements (111) associated with at least two second pixels (102) adjacent to the first pixel (102).

12. A thermal pattern sensor (100) comprising a matrix of several rows and columns of pixels (102) and a plurality of heating elements (111), each heating element (111) being associated with a pixel (102) or a group of pixels (102), and each heating element (111) being capable of heating a heat sensitive measurement element (106) of the pixel (102) or of each pixel in the group of pixels (102) independently of the other heating elements (111) during reading of the pixel (102) or at least one of the pixels in the group of pixels (102), and also comprising a control circuit capable of controlling switching the heating elements (111) on and off and making use of the method of capturing a thermal pattern according to any of the previous claims.

13. A thermal pattern sensor (100) according to claim 12, in which the heat sensitive measurement element (106) of each pixel (102) includes at least one pyroelectric capacitor formed by at least one portion of pyroelectric material (106) located between a lower electrode (108) and an upper electrode (111), in which one of the lower and upper electrodes is a read electrode of the pixel (102), in which the heating element (111) associated with each pixel (102) is capable of heating the portion of pyroelectric material (106) of the pyroelectric capacitor of the pixel (102) by the Joule effect during a measurement of the thermal pattern by the pyroelectric capacitor of said pixel (102) and is formed by the other of the lower and upper electrodes.

FIG.1

FIG.2

FIG.3

FIG.4

FIG.5

## RÉFÉRENCES CITÉES DANS LA DESCRIPTION

### Documents brevets cités dans la description

- US 4394773 A
- US 4429413 A
- US 6289114 B
- US 6091837 A
- EP 2385486 A1
- EP 0840250 A
- US 2006050935 A